(19) Europäisches Patentamt
European Patent Office
Office européen des brevets

(11) EP 2 322 227 A1

(12) **EUROPEAN PATENT APPLICATION**
published in accordance with Art. 153(4) EPC

(43) Date of publication:
**18.05.2011 Bulletin 2011/20**

(21) Application number: **09806475.1**

(22) Date of filing: **27.07.2009**

(51) Int Cl.:
***A61K 47/34*** *(2006.01)* ***A61K 9/51*** *(2006.01)*
***A61K 9/127*** *(2006.01)* ***A61K 9/107*** *(2006.01)*

(86) International application number:
**PCT/ES2009/070317**

(87) International publication number:
**WO 2010/018286 (18.02.2010 Gazette 2010/07)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **31.07.2008 ES 200802293**

(71) Applicant: **Universidade De Santiago De Compostela**
**15782 Santiago de Compostela (ES)**

(72) Inventors:
- **SOUSA-HERVES, Ana**
  **(ES)**
- **FERNANDEZ-MEGIA, Eduardo**
  **(ES)**
- **RIGUERA VEGA, Ricardo**
  **(ES)**

(74) Representative: **ABG Patentes, S.L.**
**Avenida de Burgos 16D**
**Edificio Euromor**
**28036 Madrid (ES)**

(54) **PH-SENSITIVE DENDRITIC POLYMERIC MICELLES**

(57) The present invention relates to a pH-sensitive PIC (polyion complex) type polymeric micelle formed by electrostatic interaction between a dendritic block copolymer and another polymer of opposite charge. The presence of a dendritic block copolymer confers high stability to the micelles in physiological conditions, while in the acid medium of tumor tissues (pH 6.5) or cell compartments, such as the endosome/lysosome (pH 5.0-5.5), the micelles tend to become destabilized, being able to selectively release drugs or macromolecules encapsulated therein. The micelles are **characterized by** high stability against ionic strength, dilution and temperature. They can further be lyophilized and conserved in solid state.

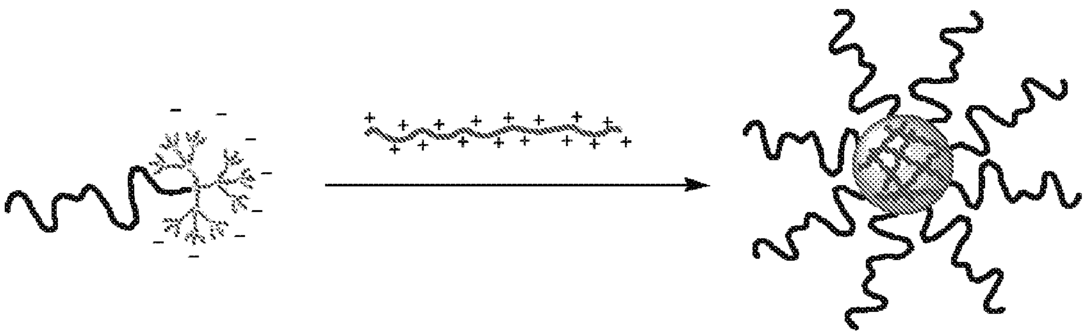

Figure 1



Printed by Jouve, 75001 PARIS (FR)

## Description

### Field of the Art

The present invention relates to a pH-sensitive polymeric micelle. More specifically, the present invention relates to a pH-sensitive polymeric micelle which contains a polymer bound to an ionic dendritic structure and has applications in nanomedicine and in diagnostic and drug delivery processes.

### Background of the Invention

In the recent years, the use of biocompatible polymers in nanomedicine has become extremely widespread. In the last decade for example, various works have been published on nanocarriers for polymeric drug delivery, including polymer-drug conjugates and polymeric micelles, the usefulness of which has been widely demonstrated. In this context, polymeric micelles have been recognized as one of the most promising carriers in the drug delivery process and in the delivery process of other bioactive species of interest in nanomedicine [Kataoka et al., J. Controlled Rel. 2001, 74, 295]. These micelles are capable of increasing the solubility, biocompatibility and the half life of drugs and other sparingly soluble species in blood.

The polymeric micelles are spontaneously formed in aqueous solution from amphiphilic block copolymers. In these conditions, the copolymers are organized such that the inside of the micelle is formed by a hydrophobic core which can serve to house drugs or other molecules of biological interest, the hydrophilic part of the copolymer being arranged towards the outside.

From the biomedical point of view, a type of very interesting polymeric micelles are the micelles known as Polyion Complex (PIC). The earliest works on this type of micelles were published by the groups of K. Kataoka [Macromolecules 1995, 28, 5294] and A. Kabanov [Bioconjugate Chem. 1995, 6, 639]. These micelles are formed by electrostatic interaction between polymers of opposite charge, having the particularity that the relationship between the charges is stoichiometric. They are nanostructures with a very low polydispersion, with zero net charge and having a size similar to that of viruses and lipoproteins (10-100 nm) which greatly facilitates their entry into target cells such as solid tumor cells.

H. Maeda [Maeda et al., Cancer Res. 1986, 46, 6387] thus highlighted for the first time the tendency for macromolecules (for example polymeric micelles) to passively accumulate in tumor tissues. This fact, known as enhanced permeability and retention effect (EPR), is considered as the most important factor for the macromolecules to access solid tumors, and it has been exploited in the context of anticancer therapy with the intention of selectively accumulating drug toxicity in the cancerous tissues, minimizing its effect on healthy tissues.

It is known today that this effect is mainly due to two factors, (i) the tumor vasculature hyperpermeability which allows the transfer of macromolecules to the tumor, (ii) poor lymphatic drainage, which causes high macromolecule retention in the tumor.

Complementarily, PIC type micelle systems the performance of which is pH-dependent [Kataoka et al., Angew. Chem. Int. Ed. 2003, 42, 4640], have recently been described. These micelles are characterized by their capacity to encapsulate a drug or macromolecule and selectively release it under the acidic conditions of tumor tissues (pH ~ 6.5) or of the endosome/lysosome (pH 5.0-5.5), without showing release in physiological conditions at neutral pH.

Biodistribution studies have further revealed that the polymeric micelles modified with hydrophilic biocompatible polymers, such as polyethylene glycol, show a prolonged circulation time in the blood stream (because they avoid recognition by the immune system [Mosqueira et al., Pharm. Res., 2001, 18, 1411]), favoring their selective accumulation in tumors.

The possibility of considerably increasing the stability of PIC micelles by means of using dendrimers has recently been disclosed. This has been attributed mainly to the high rigidity associated with the dendritic nature [Kataoka et al., Macromolecules, 2003, 36, 1304]. However, from an industrial point of view, since the synthesis of dendrimers involves long and tedious purification processes that complicate the scaling of their synthesis, a more attractive option consists of using dendritic block copolymers, wherein one of the blocks acts as a soluble polymeric support (for example polyethylene glycol), greatly facilitating the purification processes by precipitation or ultrafiltration [Riguera et al. Chem. Comm., 2008, 27, 3136].

According to the previously known and established prior art, pH-sensitive PIC type polymeric micelles obtained from dendritic block copolymers have still not been described at this time.

### Description of the Invention

#### Definitions

Dendrimer: A type of highly branched polymer in which the repeat units are organized in generations from a

focal point. Their nanometric size and multivalence make them suitable macromolecules for participating in receptor-ligand interaction processes.

Block copolymer: A copolymer is a polymer formed by two or more monomeric species. When a copolymer is formed by two blocks of different polymerized monomers, it is known as block copolymer. Block copolymers can be spatially oriented to form ordered nanostructures.

Polymeric micelles: Nanometric sized micelle formed from a block copolymer in which the two blocks have different polarities.

PIC type polymeric micelles: They are a type of polymeric micelles characterized in that their formation is not solely based on hydrophilic/hydrophobic interactions, but also on electrostatic interactions between polymers of opposite charge. They have the particularity that the relationship between charges is stoichiometric, therefore their net charge is zero. They were first described by Kataoka (1995) and were named polyion complex micelles (PIC micelles).

For the purpose of solving the problems existing in the prior art, the present invention provides the following improvements.

A type of pH-sensitive polymeric micelle containing a charged dendritic block copolymer is provided in the present invention.

One fundamental advantage of the present invention is that it greatly facilitates the overall micelle preparation process since the purification of all the steps involved in the synthesis of the block copolymer is carried out by means of a rapid precipitation or ultrafiltration. It should be pointed out that the present invention does not only greatly facilitate the synthesis of the dendritic constituent, but it also avoids the synthesis of linear block copolymer of opposite charge, allowing the use of commercial ionic linear polymers.

Another fundamental advantage of this invention is the capacity of the described pH-sensitive dendritic polymeric micelles to house other species (for example drugs or other bioactive species) therein, and to release them in the desired site (for example in the endosome/lysosome or in tumor tissues) as a result of the micelle destabilization in acid medium.

The proposed method greatly simplifies and reduces the effort necessary for obtaining a pH-sensitive dendritic polymeric micelle, with its subsequent cost reduction and suitability from the industrial point of view.

The micelles of the present invention are characterized by a high stability against ionic strength, dilution and temperature. This stability is especially pronounced when the anionic or cationic groups in the periphery of the dendritic block have a nearby aromatic ring (for example, benzoic acid or aniline). This is especially interesting when designing PIC micelles which are stable in physiological conditions.

These micelles can be lyophilized and conserved in solid state because they recover their original shape and size after being resuspended in saline buffer solution. The use of dendritic block copolymers also greatly facilitates the overall PIC micelle preparation process since all the purification processes involved in the synthesis are carried out by means of precipitation or ultrafiltration.

The present invention relates to a pH-sensitive polymeric micelle formed by electrostatic interaction between:

a) a dendritic block copolymer represented by general formula [1]

$$P\ D\ q\ (c) \quad [1]$$

wherein P is a polymer, D is a dendritic structure, q represents the number of charged atoms in the periphery of the dendritic structure which ranges between 1 and 5000, c represents a positive or negative charge, when c represents a negative charge it is due to the functional groups with pKa values between 4 and 7 in the periphery of D, and

b) a polymer M with charge c' opposite to c, wherein if c' represents a negative charge it is due to the functional groups with pKa values between 4 and 7.

The present invention also relates to a pH-sensitive polymeric micelle characterized in that the polymer P defined in claim 1 is preferably selected from linear polymers, block copolymers, copolymers, terpolymers, graft copolymers, graft terpolymers and amphiphilic copolymers.

The present invention also relates to a pH-sensitive polymeric micelle characterized in that the polymer P defined in claim 1 is preferably selected from polymers of natural origin and polymers produced by biological or chemical synthesis.

In a particular embodiment, the polymer P defined in claim 1 is a polymer of natural origin and is preferably selected from polyaminoacids, polysialic acids, hyaluronic acids and derivatives thereof, polysaccharides and derivatives thereof, chitosan and derivatives thereof, alginate and derivatives thereof, collagen and derivatives thereof, aliphatic polyesters, heparin and derivatives thereof, polycarbonates and derivatives thereof, polyhydroxyalkanoates and derivatives thereof.

In another particular embodiment, the polymer P defined in claim 1 is preferably selected from N-(2-hydroxypropyl)methacrylate (HPMA), poly(styrene-co-maleic acid/anhydride) (SMA), poly(divinyl ether maleic anhydride) (DIVEMA), poly(N-vinylpyrrolidone) (PVP), poly(N-acryloyl)morpholine (PAcM), polyethylene glycol (PEG), poly(propylene oxide) (PPO) and derivatives thereof.

In another particular embodiment, the polymer P defined in claim 1 is polyethylene glycol (PEG).

The present invention also relates to a pH-sensitive polymeric micelle characterized in that the polymer M defined in claim 1 is preferably selected from linear polymers, block copolymers, copolymers, terpolymers, graft copolymers, graft terpolymers and amphiphilic copolymers.

The present invention also relates to a pH-sensitive polymeric micelle characterized in that the polymer M defined in claim 1 is preferably selected from polymers of natural origin and polymers produced by biological or chemical synthesis.

In a particular embodiment, the polymer M defined in claim 1 is of natural origin and is preferably selected from polyaminoacids, polysialic acids, hyaluronic acids and derivatives thereof, polysaccharides and derivatives thereof, chitosan and derivatives thereof, alginate and derivatives thereof, heparin and derivatives thereof.

In another particular embodiment, the polymer M defined in claim 1 is preferably selected from polylysine, polyhistidine, polyarginine, polyglutamic acid, polyaspartic acid, chitosan and hyaluronic acid.

The present invention also relates to a pH-sensitive polymeric micelle characterized in that the dendritic structure D defined in claim 1 is a dendrimer, dendron or dendritic polymer constructed from a single or several repeat units.

In a particular embodiment, the dendritic structure D defined in claim 1 is preferably a dendron constructed from the repeat unit represented by general formula [2]

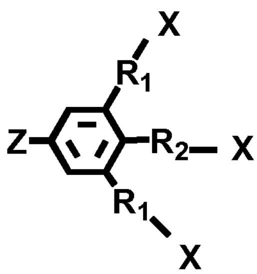

[2]

wherein Z represents a covalent bond between the repeat unit and the polymer P or a repeat unit of the previous generation. $R_1$ and $R_2$ are linear or branched chains which can be identical or different and are characterized by containing alkyl, alcohol, thiol, azide, nitrile, amine, imide, imine, cyanate, isocyanate, isothiocyanate, ether, thioether, ketone, aldehyde, ester, carboxylic acid groups or aromatic groups,;

X represents the repeat unit of the following generation or alternatively an anionic group with pKa values between 4 and 7 or a terminal group of a cationic nature.

In another particular embodiment, the dendritic structure D defined in claim 1 is preferably a dendron constructed from the repeat unit represented by general formula [2] wherein Z is preferably an amide bond;

$R_1$ and $R_2$ are identical and are selected from polyethylene glycol or oligoethylene glycol chains, they are preferably triethylene glycol;

when X is an anionic group with pKa values between 4 and 7, it is preferably selected from carboxylic acids and derivatives thereof, phosphates and derivatives thereof, phosphonates and derivatives thereof, arylphosphonic acids and derivatives thereof, phenols and derivatives thereof;

when X is a terminal group of a cationic nature, it is preferably selected from amines, polyamines, oligoamines, they are preferably spermidines, spermines, imidazoles, morpholines, ammonium salts, primary, secondary, tertiary amines or guanidinium groups.

A specific aspect of the invention relates to a pH-sensitive polymeric micelle formed by electrostatic interaction between a PEG-dendrimer block copolymer with carboxylate groups and the linear polymer poly-L-lysine.

Another specific aspect of the invention relates to a pH-sensitive polymeric micelle formed by electrostatic interaction between a PEG-dendrimer block copolymer with cationic groups and the linear polymer polyglutamic acid.

The present invention also relates to the encapsulation of a drug, protein, peptide, nucleic acid, diagnostic agent, molecule or macromolecule of any nature within the pH-sensitive polymeric micelle defined in claims 1 to 16.

The present invention also contemplates the use of a micelle, defined in claims 1 to 16, as a carrier for administering a drug or diagnostic agent.

Finally, the present invention also relates to a pharmaceutical composition comprising a pH-sensitive polymeric micelle defined in claims 1 to 16 and an active ingredient.

Figure 1 outlines a pH-sensitive polymeric micelle formed by electrostatic interaction between a PEG-dendrimer block copolymer with carboxylate groups and the linear polymer poly-L-lysine.

As has been described in detail, the present invention provides a type of pH-sensitive polymeric micelle stable in physiological conditions which can be prepared by a rapid, simple and economical method. The type of polymeric micelle described can be used for diagnostic and drug delivery processes.

The preparation of the polymeric micelles of this invention is illustrated by means of the following examples which must not be interpreted as limiting the scope of the invention in any way:

Example 1. Synthesis of the block copolymers

The dendritic unit 2 was prepared starting from methyl gallate 1 as indicated in Scheme 1. The block copolymer of generation one PEG-[G1]-$N_3$ was obtained by coupling the dendritic unit 2 with a PEG-$NH_2$ (3).

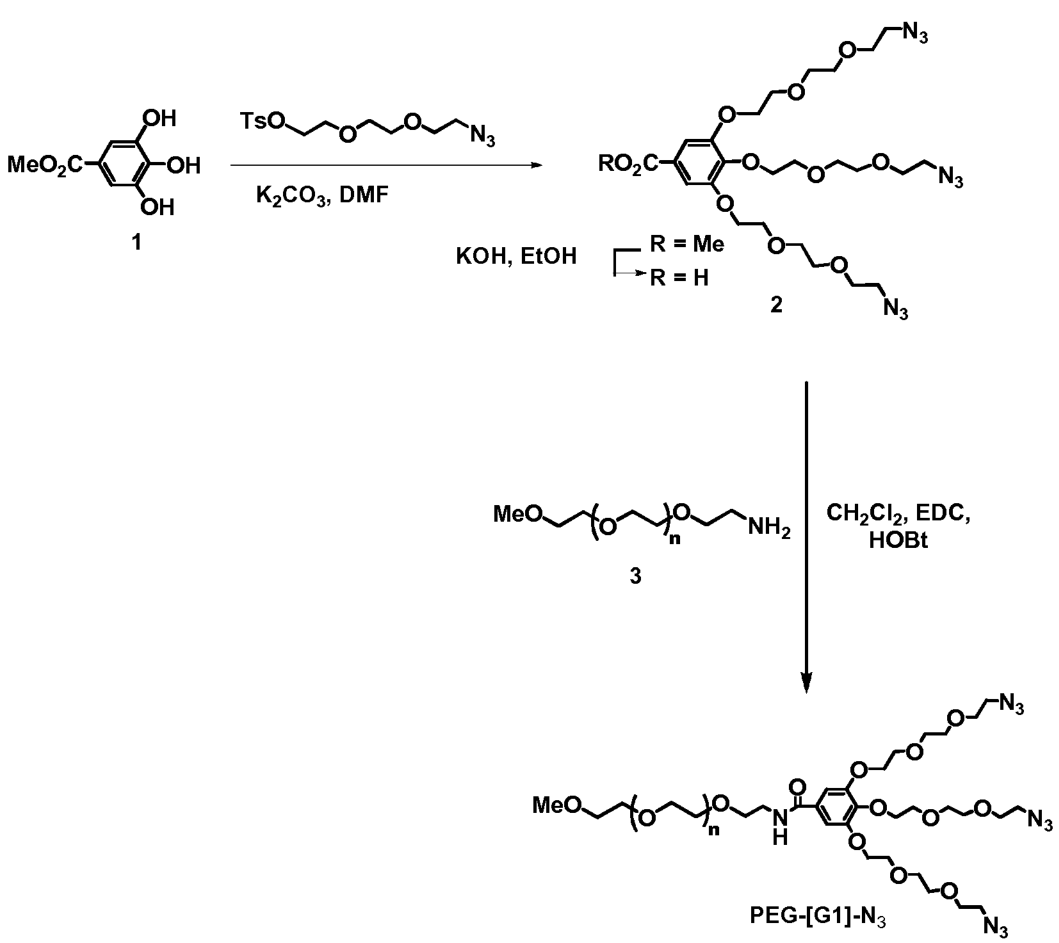


Scheme 1

The block copolymers of higher generations are obtained by reducing the terminal azide groups of the previous generation by means of catalytic hydrogenation and coupling the resulting amines with the dendritic unit 2 as shown in Scheme 2.

PEG-[G1]-N3

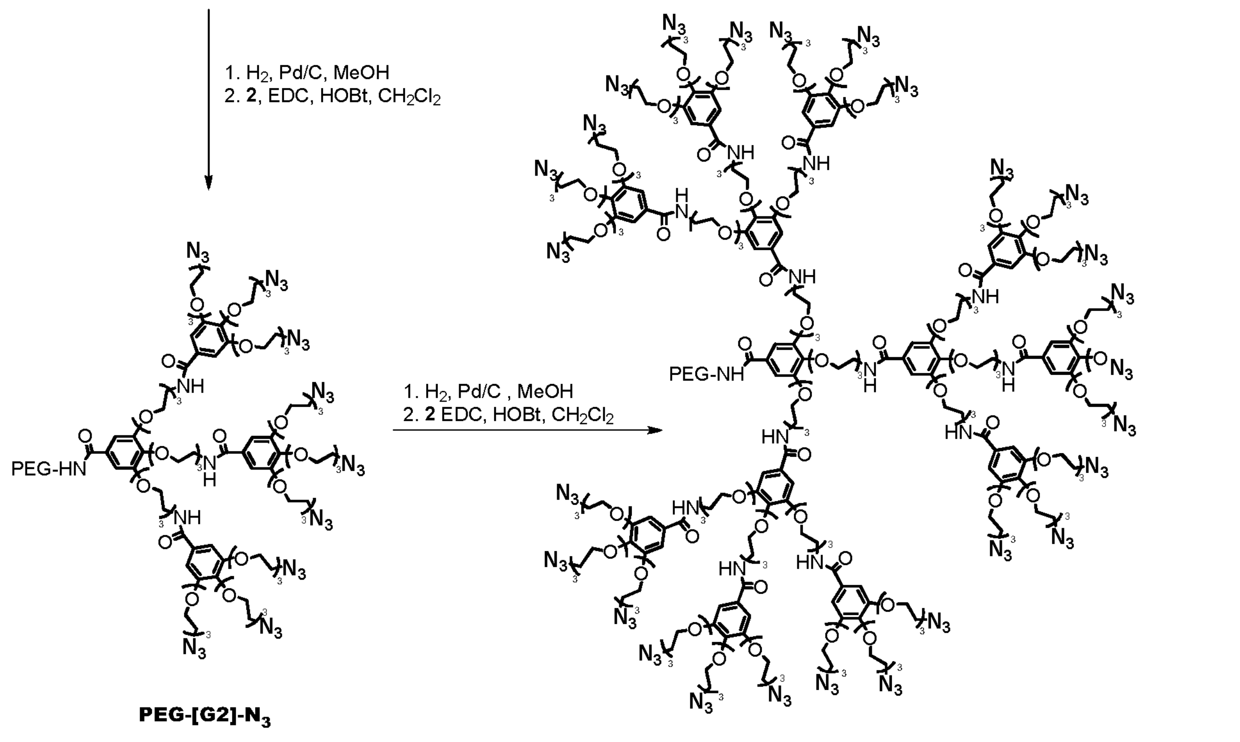


PEG-[G2]-N3

PEG-[G3]-N3

Scheme 2

Example 2. General process for anionic functionalization of the PEG-dendrimer block copolymers, (PEG-[Gn]-$N_3$)

The functionalization of the block copolymers with anionic groups was carried out by means of a Cu (I)-catalyzed azide-alkyne [3+2] cycloaddition reaction. To that end, it is necessary that the anionic ligands which are to be introduced are functionalized with terminal alkyne groups.

The PEG-dendrimer block copolymer PEG-[Gn]-$N_3$ and the anionic ligand were dissolved in a *t*-BuOH-$H_2O$ mixture (1:1). Catalytic amounts of $CuSO_4$ and sodium ascorbate were subsequently added (Scheme 3).

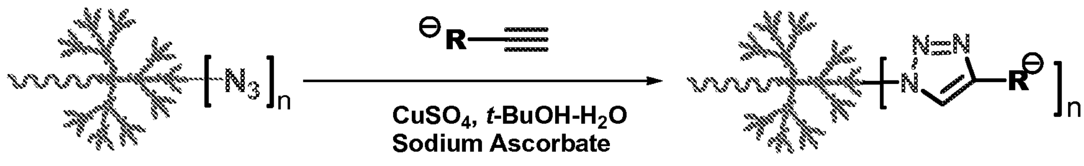


PEG-[$G_n$]-$N_3$

Scheme 3

Example 3. General process for cationic functionalization of the PEG-dendrimer block copolymers, (PEG-[Gn]-$N_3$)

Two strategies can be followed to introduce cationic groups in the periphery of the block copolymer:

1. The reduction of the terminal azides, for example by means of catalytic hydrogenation in acid medium, leads to ammonium salts of the corresponding primary amines in the periphery of the dendrimer. Scheme 4 shows how to

obtain PEG-[G3]-$NH_3^+$.

2. A Cu (I)-catalyzed azide-alkyne [3+2] cycloaddition reaction, as described in Scheme 3, but using cationic ligands functionalized with terminal alkynes.

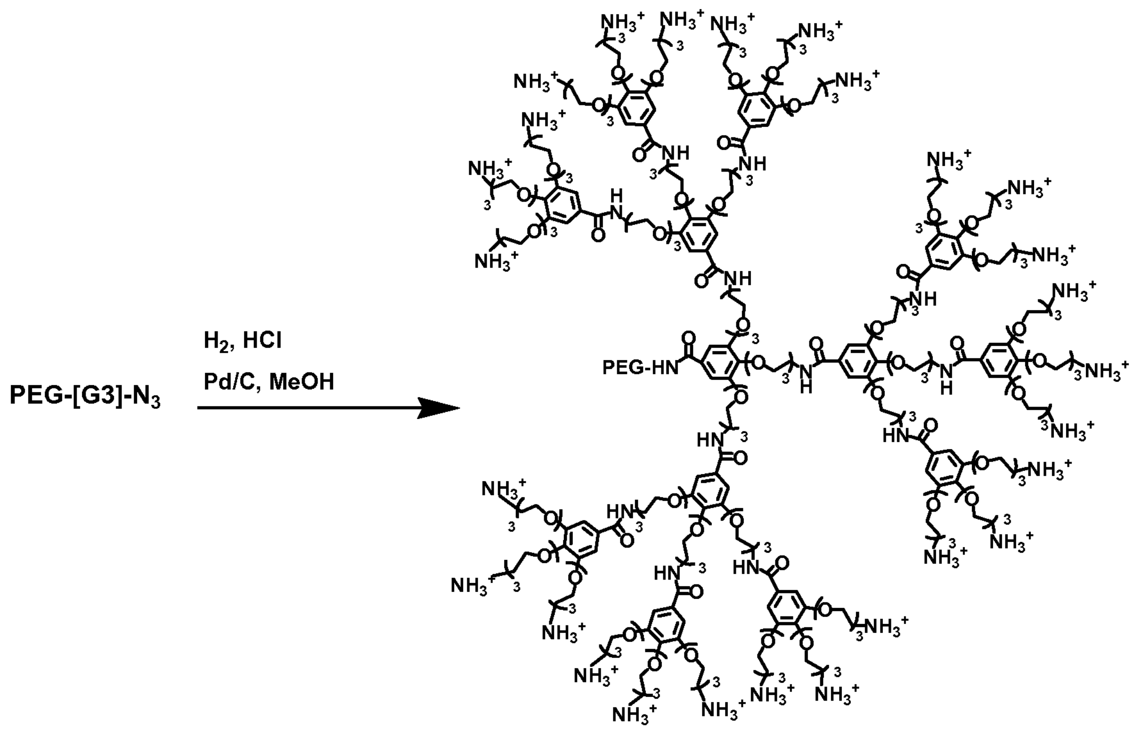


PEG-[G3]-$NH_3^+$

Scheme 4

Example 4. Preparation of pH-sensitive polymeric micelles from a block copolymer functionalized with carboxylate groups and a polycationic linear polymer

The block copolymer PEG-[G3]-$CO_2^-$ (obtained from PEG-$NH_2$, $M_n$ 5219) was dissolved in PBS/NaOH pH 12. Poly-L-lysine ($M_n$ 12400) was dissolved separately in PBS pH 4.95. The two solutions were filtered and mixed in a charge stoichiometric ratio to give rise to PIC type micelles with a concentration of 1 mg/ml and pH 7.4. After 24 hours under magnetic stirring, the resulting solution was filtered and was analyzed by DLS (Dynamic Light Scattering). The DLS measurements confirmed the existence of polymeric micelles with a very low polydispersion, a size of 24 nm and a near zero z-potential. These data were corroborated by TEM (Transmission Electron Microscopy) and by AFM (Atomic Force Microscopy).

Example 5. Preparation of pH-sensitive polymeric micelles from a cationic block copolymer and a polycarboxylated linear polymer

The block copolymer PEG-[G3]-$NH_3^+$ (obtained from PEG-$NH_2$, $M_n$ 5219) was dissolved in a PBS buffer pH 4.95. Polyglutamic acid (PGA) ($M_v$ 14500) was dissolved separately in PBS pH 9.0. The two solutions were filtered and mixed in a charge stoichiometric ratio to give rise to PIC type micelles with a concentration of 1 mg/ml and pH 7.4. After 24 hours under magnetic stirring, the resulting solution was filtered and analyzed by DLS (Dynamic Light Scattering). The DLS measurements confirmed the existence of polymeric micelles with very low polydispersion, a size of 23 nm and a near zero z-potential. These data were corroborated by TEM (Transmission Electron Microscopy) and AFM (Atomic Force Microscopy).

Example 6. Stability of the micelles against pH

Several solutions of the PIC micelles described in examples 4 and 5 were prepared at different pHs for testing the stability of the micelles against pH.

After 24 hours the solutions were analyzed by DLS, a clear destabilization of the micelles at pH ≤ 5.5 being observed.

Examples 7 and 8 illustrate pH-sensitive PIC micelles prepared from dendritic block copolymers which contain ionizable groups near an aromatic ring in the periphery. These micelles are more stable against higher ionic strength and temperature as compared to micelles obtained from block copolymers which lack said aromatic rings. The micelles of examples 7 and 8 have been characterized by means of Dynamic Light Scattering (DLS), Gel Permeation Chromatography (GPC), Atomic Force Microscopy (AFM) and Transmission Electron Microscopy (TEM). The stability of these micelles in physiological conditions (150 mM NaCl, 37°C) and against the ionic strength and lyophilization has been studied by means of DLS. The sensitivity of said micelles against pH has been analyzed by means of DLS and Nuclear Magnetic Resonance (NMR). Cytotoxicity studies on these micelles have also been performed in two cell lines.

Example 7. Preparation of pH-sensitive polymeric micelles from a block copolymer functionalized with benzoate groups and the polycationic linear polymer Poly-L-lysine (PLL, $M_n$ 12400).

The block copolymer PEG-[G3]-Ph-$CO_2^-$ (obtained from PEG-$NH_2$, $M_n$ 5219) was dissolved in PBS/NaOH pH 12. PLL ($M_n$ 12400) was dissolved separately in PBS pH 4.95. The two solutions were filtered and mixed in a charge stoichiometric ratio to give rise to PIC type micelles with a concentration of 1 mg/mL and pH 7.4. After 24 hours under magnetic stirring, the resulting solution was filtered and analyzed by DLS (Figure 2: Formation of a pH-sensitive PIC micelle from a block copolymer with benzoate groups and DLS histogram). The DLS measurements confirmed the existence of polymeric micelles with very low polydispersion (0.09), a size of 35 nm and a near zero z-potential. These data were corroborated by AFM (Figure 3: AFM of pH-sensitive PIC micelles formed from a block copolymer with benzoate groups) and TEM (Figure 4: TEM of pH-sensitive PIC micelles formed from a block copolymer with benzoate groups).

GPC: The quantitative formation of the PIC micelles from their constituents was demonstrated by means of GPC. Figure 5 [Elugram of pH-sensitive PIC micelles formed from a block copolymer with benzoate groups and PLL (PBS pH 7.4, left curve) and from the block copolymer with benzoate groups (PBS pH 7.4, right curve)] shows the elugram of a pH-sensitive PIC micelles solution formed from the block copolymer with benzoate groups in the periphery and PLL in which signals of the block copolymer are not observed.

Stability in physiological conditions and against ionic strength, dilution and lyophilization: The stability of these PIC micelles in physiological conditions was determined by means of adding NaCl to the original solution of micelles until a concentration equal to 150 mM. The solution was subsequently heated to 37°C and the solution of micelles was analyzed by means of DLS, no change in the size or any other sign of destabilization being observed. It was proven that these micelles are stable up to a saline concentration of 500 mM.

The stability of the micelles against dilution was determined by DLS. No change was observed up to a concentration of 0.2 mg/mL, which represents the limit of detection of the technique for the particle size.

The stability of the micelles against lyophilization was proven by DLS after resuspension as no change in the size of the micelles is observed.

Sensitivity against pH: For studying the sensitivity of these micelles against pH, HCl was added to a solution thereof in PBS pH 7.4 until pH 5.2 is obtained. The resulting solution was stirred at room temperature for 12 hours and was subsequently analyzed by DLS. The DLS measurements indicated a clear destabilization of the micelles, the appearance of new species and a great increase in polydispersion, PDI 0.5, being observed (Figure. 6: DLS histogram at pH 5.2 of pH-sensitive PIC micelles formed from a block copolymer with benzoate groups).

The sensitivity of these micelles to pH was also studied by means of NMR. To that end, different aliquots the pH of which was adjusted to 6.0, 5.5, 5.0 and 4.0 were prepared from a solution of PIC micelles of pH 7.4. Solutions of the block copolymer PEG-[G3]-Ph-$CO_2^-$ and of the polycationic PLL at pH 7.4 and pH 4.0 were also prepared. After 12 hours under stirring at room temperature, the $^1$H spectrums of all the solutions were recorded.

The comparison of normalized $^1$H spectrums revealed a reduction between 80-100% in the intensity of the signals corresponding to the protons of the dendritic block and PLL which form the compact core of the micelle at pH 7.4. However, the signals corresponding to the protons of the PLL recover their intensity at pH 5.0, demonstrating destabilization of the micelles. Simultaneously, after reducing the pH, the signals corresponding to the protons in the periphery of the dendritic block disappear completely, presumably due to the lower solubility thereof after protonation.

Cytotoxicity: Cytotoxicity studies of the micelles were performed in two cell lines, MCF-7 (human mammary carcinoma cells) and NCI-H460 (human lung carcinoma cells). To that end, a solution of micelles was prepared at a concentration of 6 mg/mL and the inhibition on cell growth was evaluated using a system based on cell staining by crystal violet and subsequent acetylation. The PIC micelles presented such reduced inhibition on cell growth that it was not

possible to plot the cell growth inhibition curves.

Example 8: Preparation of pH-sensitive polymeric micelles from a block copolymer functionalized with aniline groups and the polycarboxylated linear polymer Polyglutamic acid (PGA, $M_v$ 14500).

The block copolymer PEG-[G3]-Ph-$NH_3^+$ (obtained from PEG-$NH_2$, $M_n$ 5219) was dissolved in a PBS buffer pH 3.00. PGA ($M_v$ 14500) was dissolved separately in PBS pH 9.0. The two solutions were filtered and mixed in a charge stoichiometric ratio to give rise to PIC type micelles with a concentration of 1 mg/mL and pH 7.4. After 24 hours under magnetic stirring, the resulting solution was filtered and analyzed by DLS (Figure 7: Formation of a pH-sensitive PIC micelle from a block copolymer with aniline groups and DLS histogram). The DLS measurements confirmed the existence of polymeric micelles with low polydispersion (0.16), a size of 39 nm and a near zero z-potential. These data were corroborated by TEM (Figure 8: TEM of pH-sensitive PIC micelles formed from a block copolymer with aniline groups) and AFM.

GPC: The PIC micelles formed from block copolymer with aniline groups and PGA show a lower elution time than the block copolymer [Figure 9: Elugram of pH-sensitive PIC micelles formed from a block copolymer with aniline groups and PGA (PBS pH 7.4, left curve) and from the block copolymer with aniline groups (PBS pH 7.4, right curve)].

Stability in physiological conditions: The stability of these PIC micelles in physiological conditions was determined by means of adding NaCl to the original solution of micelles until a concentration equal to 150 mM. The solution was subsequently heated to 37° C and the solution of micelles was analyzed by means of DLS, no change in the size or any other sign of destabilization being observed.

Sensitivity against pH: For studying the sensitivity of these micelles against pH, HCl was added to a solution thereof in PBS pH 7.4, 150 mM NaCl until obtaining pH 5.0. The resulting solution was stirred at room temperature for 12 hours, and was subsequently analyzed by DLS. The DLS measurements indicated a clear destabilization of the micelles, the appearance of new species and a great increase in polydispersion, PDI 0.6, being observed (Figure 10: DLS histogram at pH 5.0 of pH-sensitive PIC micelles formed from a block copolymer with aniline groups).

Cytotoxicity: Cytotoxicity studies of the micelles were performed in two cell lines, MCF-7 (human mammary carcinoma cells) and NCI-H460 (human lung carcinoma cells). To that end, a solution of micelles was prepared at a concentration of 6 mg/mL and the inhibition on cell growth was evaluated using a system based on cell staining by crystal violet and subsequent acetylation. The PIC micelles presented reduced inhibition on cell growth that it was not possible to plot the cell growth inhibition curves.

## Claims

1. A pH-sensitive polymeric micelle formed by electrostatic interaction between:

   a) a dendritic block copolymer represented by general formula [1]

   $$P\ D\ q\ (c) \quad [1]$$

   wherein P is a polymer, D is a dendritic structure, q represents the number of charged atoms in the periphery of the dendritic structure which ranges between 1 and 5000, c represents a positive or negative charge, when c represents a negative charge it is due to the functional groups with pKa values between 4 and 7 in the periphery of D, and

   b) a polymer M with charge c' opposite to c, wherein if c' represents a negative charge it is due to the functional groups with pKa values between 4 and 7.

2. The polymeric micelle according to claim 1, wherein the polymer P is preferably selected from linear polymers, block copolymers, copolymers, terpolymers, graft copolymers, graft terpolymers and amphiphilic copolymers.

3. The polymeric micelle according to claim 1, wherein the polymer P is preferably selected from polymers of natural origin or polymers produced by biological or chemical synthesis.

4. The polymeric micelle according to claim 3, wherein the polymer P is of natural origin and is preferably selected from polyaminoacids, polysialic acids, hyaluronic acids and derivatives thereof, polysaccharides and derivatives thereof, chitosan and derivatives thereof, alginate and derivatives thereof, collagen and derivatives thereof, aliphatic

polyesters, heparin and derivatives thereof, polycarbonates and derivatives thereof, polyhydroxyalkanoates and derivatives thereof.

**5.** The polymeric micelle according to claims 1, 2, 3 and 4, wherein the polymer P is selected from polyesters, polycarbonates, polyamides, poly(ester-amides), polyanhydrides, polyurethanes, poly(ester-urethanes), polyhydroxyalkanoates and derivatives thereof.

**6.** The polymeric micelle according to the previous claims, wherein the polymer P is preferably selected from N-(2-hydroxypropyl)methacrylate (HPMA), poly(styrene-co-maleic acid/anhydride) (SMA), poly(divinyl ether maleic anhydride) (DIVEMA), poly(N-vinylpyrrolidone) (PVP), poly(N-acryloyl)morpholine (PAcM), polyethylene glycol (PEG), poly(propylene oxide) (PPO) and derivatives thereof.

**7.** The polymeric micelle according to the previous claims, wherein the polymer P is preferably polyethylene glycol (PEG).

**8.** The polymeric micelle according to claim 1, wherein the polymer M is preferably selected from linear polymers, block copolymers, copolymers, terpolymers, graft copolymers, graft terpolymers and amphiphilic copolymers.

**9.** The polymeric micelle according to claim 1, wherein the polymer M is preferably selected from polymers of natural origin or polymers produced by biological or chemical synthesis.

**10.** The polymeric micelle according to claim 9, wherein the polymer of natural origin is preferably selected from polyaminoacids, polysialic acids, hyaluronic acids and derivatives thereof, polysaccharides and derivatives thereof, chitosan and derivatives thereof, alginate and derivatives thereof, heparin and derivatives thereof.

**11.** The polymeric micelle according to claims 1, 9 and 10, wherein the polymer M is preferably selected from polylysine, polyhistidine, polyarginine, polyglutamic acid, polyaspartic acid, chitosan and hyaluronic acid.

**12.** The polymeric micelle according to claim 1, wherein the dendritic structure D is a dendrimer, dendron or dendritic polymer constructed from the same or different repeat units.

**13.** The polymeric micelle according to claims 1 and 12, wherein D is preferably a dendron constructed from the repeat unit represented by general formula [2]

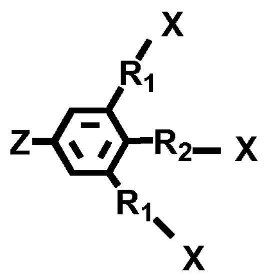

[2]

wherein Z represents a covalent bond between the repeat unit and the polymer P or the repeat unit of the previous generation,
$R_1$ and $R_2$ are linear or branched chains which can be identical or different and are **characterized by** containing alkyl, alcohol, thiol, azide, nitrile, amine, imide, imine, cyanate, isocyanate, isothiocyanate, ether, thioether, ketone, aldehyde, ester, carboxylic acid groups or aromatic groups,
X represents the repeat unit of the following generation or alternatively an anionic group with pKa values between 4 and 7 or a terminal group of a cationic nature.

**14.** The polymeric micelle according to claim 13, wherein Z is preferably an amide bond;
$R_1$ and $R_2$ are identical and are selected from polyethylene glycol or oligoethylene glycol chains, they are preferably

triethylene glycol;
when X is an anionic group with pKa values between 4 and 7, it is preferably selected from carboxylic acids and derivatives thereof, phosphates and derivatives thereof, phosphonates and derivatives thereof, arylphosphonic acids and derivatives thereof, phenols and derivatives thereof;
when X is a terminal group of a cationic nature, it is preferably selected from amines, polyamines, oligoamines, they are preferably spermidines, spermines, imidazoles, morpholines, ammonium salts, primary, secondary, tertiary amines or guanidinium groups.

**15.** The polymeric micelle according to claim 1, **characterized in that** P is polyethylene glycol, D contains carboxylate groups in its periphery and M is poly-L-lysine.

**16.** The polymeric micelle according to claim 1, **characterized in that** P is PEG, D contains positively charged amino groups in its periphery and M is polyglutamic acid.

**17.** The polymeric micelle according to the previous claims, **characterized in that** a drug, a diagnostic agent, a molecule or a macromolecule of any nature is encapsulated therein.

**18.** Use of a micelle according to claims 1 to 16 as a carrier for administering a drug or diagnostic agent.

**19.** A pharmaceutical composition comprising a micelle according to claims 1 to 16 and an active ingredient.

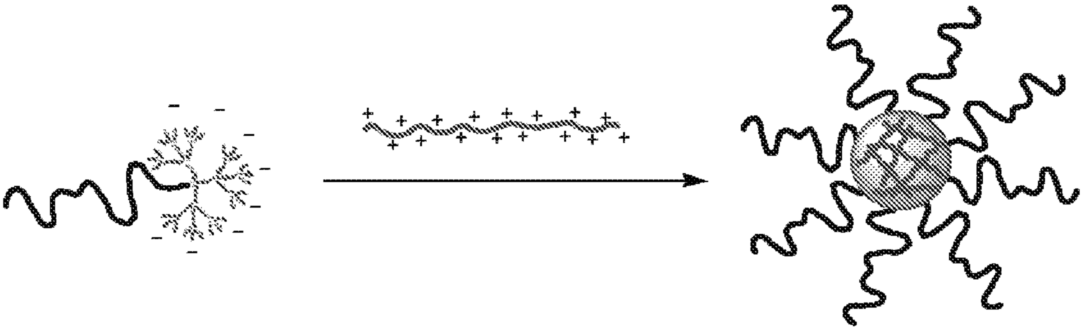

Figure 1

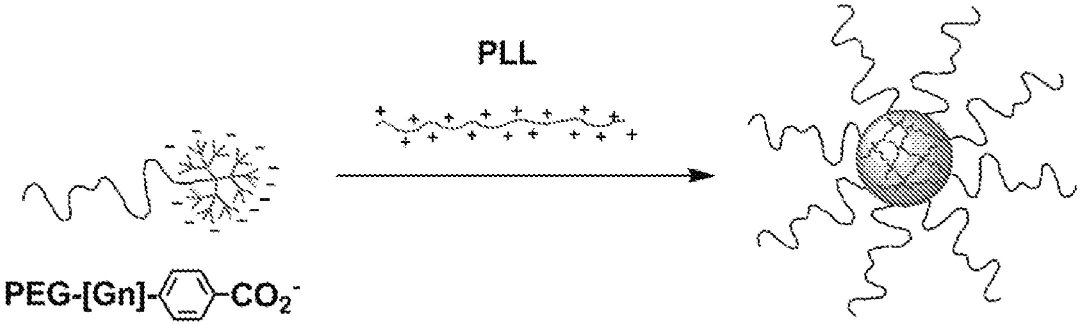


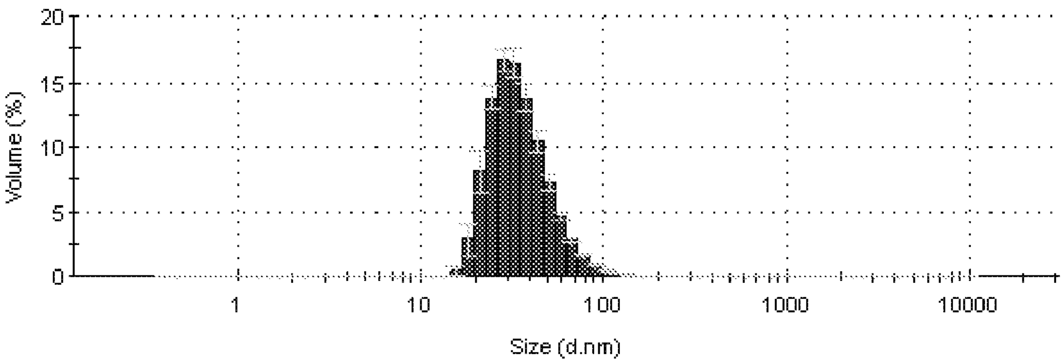


Figure 2

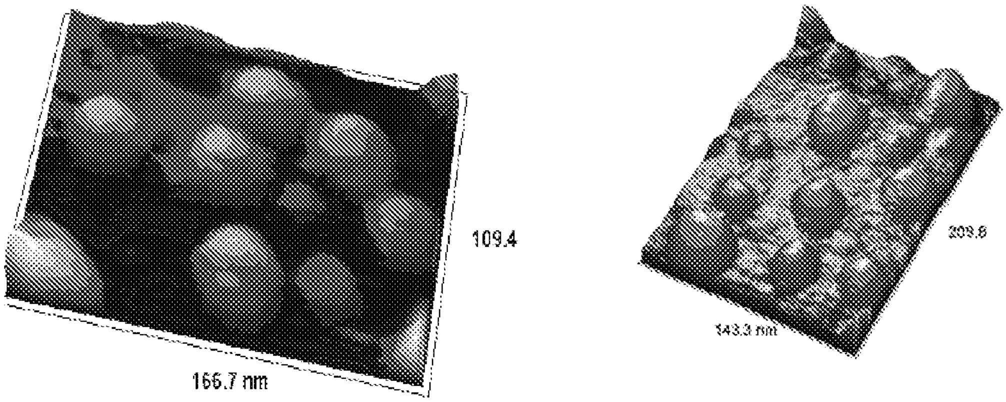


Figure 3

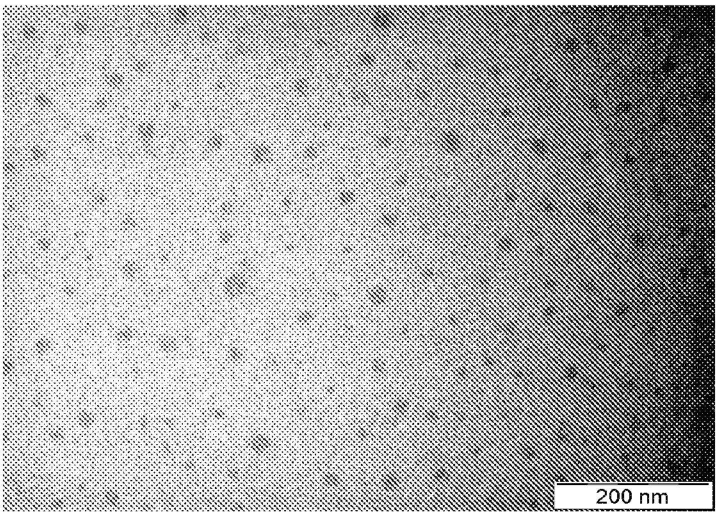


Figure 4

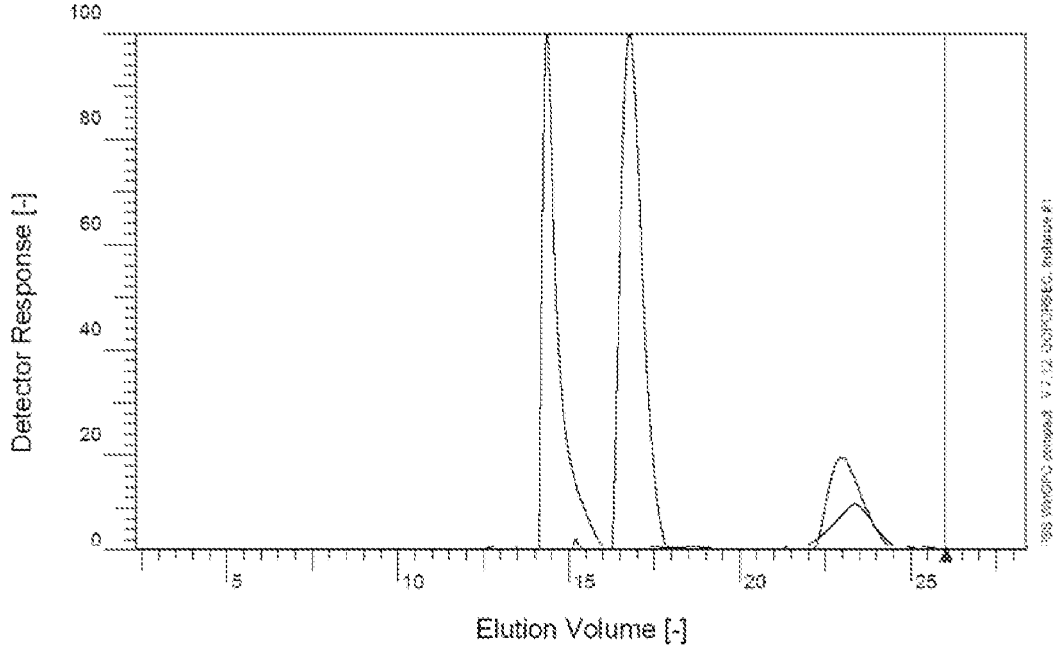


Figure 5

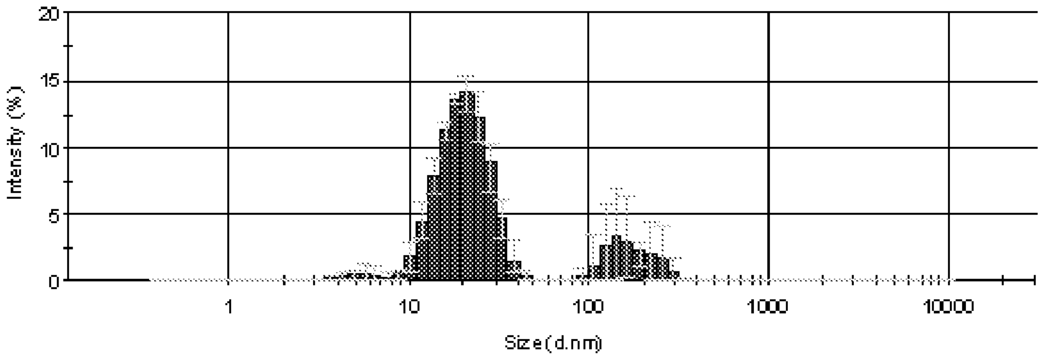


Figure 6

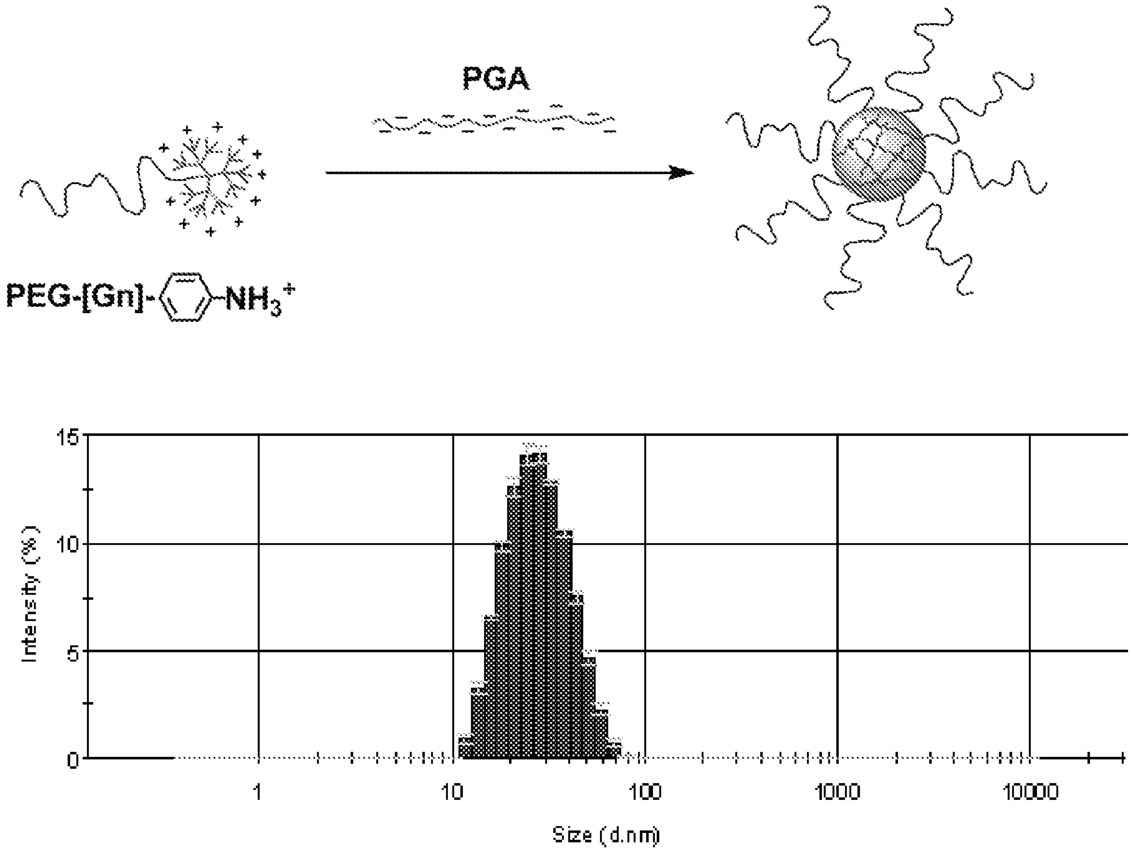


Figure 7

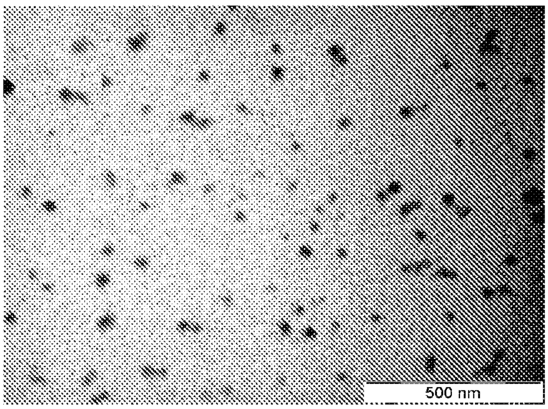


Figure 8

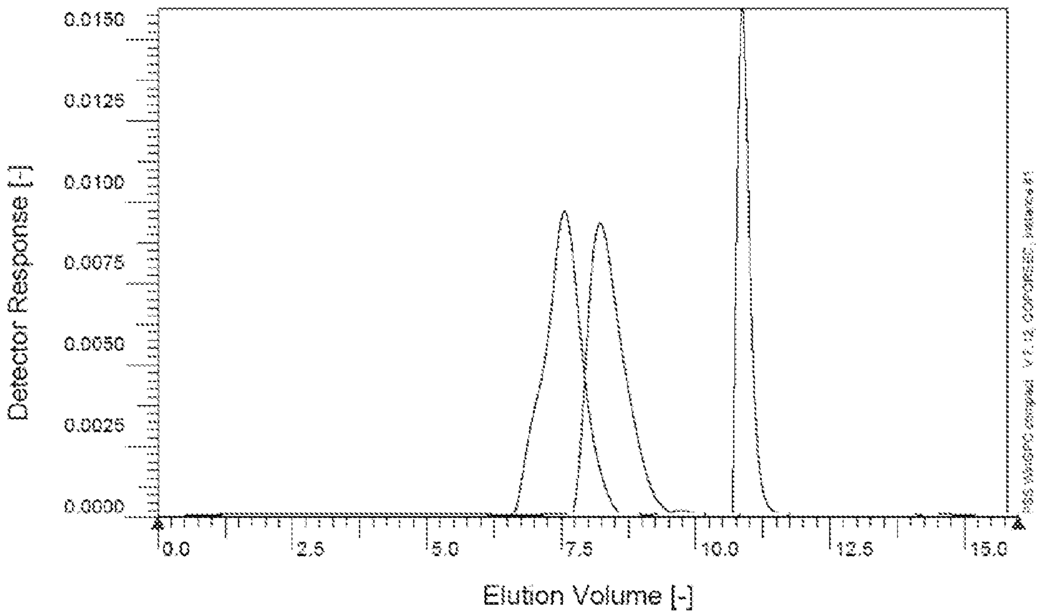


Figure 9

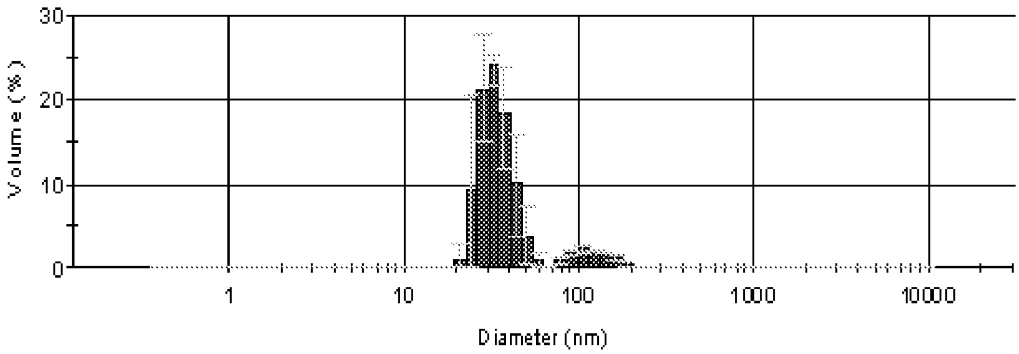


Figure 10

## INTERNATIONAL SEARCH REPORT

International application No.
PCT/ ES 2009/070317

A. CLASSIFICATION OF SUBJECT MATTER

see extra sheet

According to International Patent Classification (IPC) or to both national classification and IPC

B. FIELDS SEARCHED

Minimum documentation searched (classification system followed by classification symbols)

A61K

Documentation searched other than minimum documentation to the extent that such documents are included in the fields searched

Electronic data base consulted during the international search (name of data base and, where practicable, search terms used)

INVENES, EPODOC, CAS, WPI, MEDLINE, EMBASE, BIOSIS

C. DOCUMENTS CONSIDERED TO BE RELEVANT

| Category* | Citation of document, with indication, where appropriate, of the relevant passages | Relevant to claim No. |
|---|---|---|
| A | JANG, WOO-DONG et al.; Polyon complex micelles for photodynamic therapy: Incorporation of dendritic photosensitizer excitable at long wavelenght relevant to improved tissue-penetrating property; Journal of Controlled Release (2006), volume 113, número 1, pages 73-79; ISSN 0168-3659. | 1-19 |
| A | ZHANG, GUO-DONG et al.; Polyon complex micelles entrapping dendrimer porphyrin: effective photosensitizer for photodynamic therapy of cancer; Journal of Controlled Release (2003), volume 93, número 2, pages 141-150; ISSN 0168-3659. | 1-19 |

☒ Further documents are listed in the continuation of Box C. ☐ See patent family annex.

* Special categories of cited documents:

- "A" document defining the general state of the art which is not considered to be of particular relevance.
- "E" earlier document but published on or after the international filing date
- "L" document which may throw doubts on priority claim(s) or which is cited to establish the publication date of another citation or other special reason (as specified)
- "O" document referring to an oral disclosure use, exhibition, or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other documents , such combination being obvious to a person skilled in the art
- "&" document member of the same patent family

| Date of the actual completion of the international search | Date of mailing of the international search report |
|---|---|
| 30.November.2009 (30.11.2009) | (02/12/2009) |
| Name and mailing address of the ISA/<br>O.E.P.M.<br>Paseo de la Castellana, 75 28071 Madrid, España.<br>Facsimile No. 34 91 3495304 | Authorized officer<br>N. Vera Gutiérrez<br>Telephone No. +34 91 349 55 44 |

Form PCT/ISA/210 (second sheet) (July 2008)

**INTERNATIONAL SEARCH REPORT**

International application No.
PCT/ES 2009/070317

C (continuation). DOCUMENTS CONSIDERED TO BE RELEVANT

| Category* | Citation of documents, with indication, where appropriate, of the relevant passages | Relevant to claim No. |
|---|---|---|
| A | GILLIES, ELIZABETH R.et al.; Stimuli-responsive supramolecular assemblies of liner-dendritic copolymers; J.Am.Chem.Soc. (2004), 126, pages 11936-11943. | 1-19 |
| A | GILLIES, ELIZABETH R. et al.; Dendrimers and dendritic polymers in drug delivery; Drug Discovery Today, January 2005, volume 10, número 1, pages 35-43; ISSN 1359-6446. | 1-19 |

Form PCT/ISA/210 (continuation of second sheet) (July 2008)

**INTERNATIONAL SEARCH REPORT**

International application No.

PCT/ ES 2009/070317

CLASSIFICATION OF SUBJECT MATTER

***A61K 47/34*** (2006.01)
***A61K 9/51*** (2006.01)
***A61K 9/127*** (2006.01)
***A61K 9/107*** (2006.01)

Form PCT/ISA/210 (extra sheeet) (July 2008)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **Kataoka et al.** *J. Controlled Rel.,* 2001, vol. 74, 295
- **K. Kataoka.** *Macromolecules,* 1995, vol. 28, 5294
- **A. Kabanov.** *Bioconjugate Chem.,* 1995, vol. 6, 639
- **Maeda et al.** *Cancer Res.,* 1986, vol. 46, 6387
- **Kataoka et al.** *Angew. Chem. Int. Ed.,* 2003, vol. 42, 4640
- **Mosqueira et al.** *Pharm. Res.,* 2001, vol. 18, 1411
- **Kataoka et al.** *Macromolecules,* 2003, vol. 36, 1304
- **Riguera et al.** *Chem. Comm.,* 2008, vol. 27, 3136